(19) Europäisches Patentamt
European Patent Office
Office européen des brevets

(11) Veröffentlichungsnummer: **0 485 890 A2**

(12) # EUROPÄISCHE PATENTANMELDUNG

(21) Anmeldenummer: **91119002.3**

(22) Anmeldetag: **07.11.91**

(51) Int. Cl.[5]: **A61K 31/41**, A61K 31/415, A61K 31/495, A61K 31/47, A61K 31/44, A61K 31/42, A61K 31/425

(30) Priorität: **16.11.90 DE 4036642**

(43) Veröffentlichungstag der Anmeldung: **20.05.92 Patentblatt 92/21**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**W-6230 Frankfurt am Main 80(DE)**

(72) Erfinder: **Kottmann, Hariolf, Dr.**
**Am alten Birnbaum 6**
**W-6238 Hofheim am Taunus(DE)**
Erfinder: **Hänel, Heinz, Dr.**
**Taunusstrasse 148**
**W-6370 Oberursel(DE)**
Erfinder: **Blume, Ernst, Dr.**
**Rossertstrasse 20**
**W-6239 Kriftel/Taunus(DE)**
Erfinder: **Kirsch, Reinhard, Dr.**
**Johannesallee 18**
**W-6230 Frankfurt am Main(DE)**

(54) **Verwendung von 2,3-disubstituierten 1-Azolyl-propanen als Wachstumshemmer der pathogenen Phase dimorpher Hefezellen.**

(57) Verbindungen der Formel I

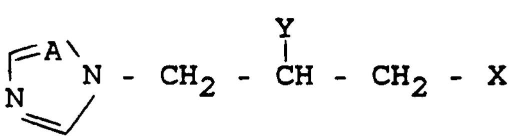

I

mit
A gleich CH, N;
X gleich -NR(1)R(2) -O-Phenyl-, O-Benyzl-, S-Phenyl, -S-Benzyl, O-N = R(3)R(4),
mit R(1) gleich (Cyclo)-Alk(en)yl, (Alkyl-)Phenyl, (Alkyl-)Hetaryl und anderen und R(2) gleich Wasserstoff oder R-(1); oder R(1) und R(2) gemeinsam eine Kette $(CH_2)_{4-6}$;
R(3) gleich Phenyl, Biphenylyl, Phenylmethylphenyl, Phenyl-Z-Phenyl, Benzyl, Alkyl und Z gleich Sauerstoff, Schwefel, Sulf(i)(o)nyl und R(3) gleich Alk(en)(in)yl, Phenyl, Benzyl
Y gleich OH, SH, Hal, Z-R(5) mit R(5) gleich (cyclo)-Alk(en)yl, Alkylphenyl, Alkyl, Hetaryl, Biphenylyl, Phenyl-Z-Phenyl, Phenylmethyl-Phenyl, Naphthyl
eignen sich hervorragend zur Prophylaxe und zur Behandlung von systemischen Mykosen.

EP 0 485 890 A2

Die Erfindung betrifft die Verwendung von 2,3-disubstituierten 1-Azolyl-propanen und solche Verbindungen enthaltende pharmazeutische Zusammensetzungen als Pharmazeutika, insbesondere als Wachstumshemmer der pathogenen Phase dimorpher Hefezellen, wobei die Verwendung der Verbindungen sowohl in der Prophylaxe als auch in der Therapie stattfinden kann.

2,3-disubstituierte 1-Azolyl-propane sind bekannt. Beschrieben sind antithrombotische Wirkung (EP 62-918) sowie fungizide, antibakterielle und antiprotozoale Wirkung (US 4 036 970, US 4 036 973, US 4 036 974).

Aufgrund der für eine ausreichend fungizide Wirksamkeit erforderlichen hohen Dosierung der erfindungsgemäßen verwendeten Verbindungen und den damit verbundenen Nebenwirkungen erreichte bisher keine Verbindung aus dieser Substanzklasse therapeutische Bedeutung.

Von einigen strukturell verwandten β-Hydroxyethylaminen, die als Betablocker verwendet werden, ist bekannt, daß sie eine allerdings geringe Hemm-Wirkung auf die lysomale Phospholipase A1 von Säugern besitzen (vgl. K.Y. Hostetler et al., Biochem. Pharmacology 34, 521-524 (1985)).

Es wurde nun überraschenderweise gefunden, daß 2,3-disubstituierte 1-Azolyl-propane, in Abhängigkeit von der Struktur der jeweiligen Substituenten, wesentlich stärkere Hemmstoffe der Exoenzyme von Hefen sind als die obengenannten Betablocker und daß diese ausgeprägte Wirksamkeit insbesondere dann beobachtet wird, wenn die verabreichte Dosis der erfindungsgemäßen Verbindungen deutlich niedriger liegt, als die bei fungizider Wirkung ermittelten MFK-Werte (minimale fungizide Konzentration) der 2,3-disubstituierten 1-Azolyl-propanen.

Damit konnte überraschenderweise gezeigt werden, daß bei Verabreichung der erfindunggemäß verwendeten Verbindungen in Dosen, die deutlich unterhalb der MFK-Werte liegen, zwar eine wachstumshemmende, jedoch keine pilztötende Wirkung, die meist mit toxikologischen Nebenwirkungen behaftet ist, erzielt wird.

Damit eignen sich die erfindungsgemäß verwendeten Verbindungen speziell zur schonenden Prophylaxe, aber auch zu einer toxikologisch geringer belastenden Therapie schwerer systemischer Mykosen, insbesondere solcher, die durch Candida albicans hervorgerufen sind.

Die Erfindung betrifft daher die Verwendung von 2,3-disubstituierten 1-Azolyl-propanen sowie diese Verbindungen enthaltende pharmazeutische Zusammensetzungen der Formel I

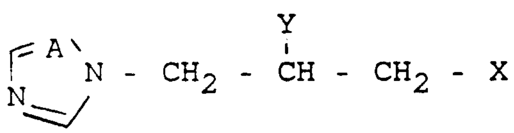

I

zur Behandlung von, insbesondere jedoch zur Vorbeugung gegen Pilzerkrankungen; in den Verbindungen bedeuten:

A — CH, N

X — -NR(1)R(2), -O-Phenyl, -O-Benzyl, S-Phenyl, S-Benzyl, wobei Phenyl und Benzyl unsubstituiert oder mit ein bis drei Substituenten aus der Reihe F, Cl, Br, O-($C_1$-$C_4$)-Alkyl, ($C_1$-$C_4$)-Alkyl, $CF_3$ substituiert ist), -O-N = CR(3)R(4), worin

R(1) — ($C_1$-$C_{15}$)-Alkyl, ($C_2$-$C_{15}$)-Alkenyl, ($C_2$-$C_{15}$)-Alkinyl, ($C_3$-$C_{15}$)-Alken-in-yl, ($C_3$-$C_8$)-Cycloalkyl, Phenyl, ($C_1$-$C_4$)-Alkyl-Phenyl, Hetaryl, ($C_1$-$C_4$)-Alkyl-Hetaryl, Biphenylyl, Phenyl-Z-Phenyl, Phenyl-methyl-Phenyl, Naphthyl ist, wobei die aufgeführten aromatischen und heteroaromatischen Ringsysteme unsubstituiert oder mit ein bis drei Substituenten - gleich oder verschieden - aus der Reihe F, Cl, Br, $CF_3$, CN, $NO_2$, ($C_1$-$C_{10}$)-Alkyl, ($C_2$-$C_{10}$)-Alkenyl, Z-($C_1$-$C_{10}$)-Alkyl, Z-($C_2$-$C_{10}$)-Alkenyl, Z-($C_3$-$C_8$)-Cycloalkyl substituiert sind, Z für Sauerstoff, Schwefel, Sulfinyl, Sulfonyl steht und das heteroaromatische Ringsystem ein bis fünf Kohlenstoffatome sowie ein bis drei Heteroatome - gleich oder verschieden - aus der Reihe N, O, S enthält (z.B. seien als Heteroaromaten genannt Thiophen, Pyridin, Furan, Oxazol, Isoxazol, Thiazol, Isthiazol)

und

R(2) — Wasserstoff bedeutet oder wie R(1) definiert ist, wobei R(1) und R(2) gleich oder verschieden sind,

oder

R(1) und R(2) — gemeinsam eine Kette von $(CH_2)_{4-6}$-Einheiten bilden, in welcher eine ($CH_2$)-Einheit durch ein Sauerstoff-, Schwefel- oder Stickstoffatom ersetzt sein kann und wobei das zusätzliche N-Atom als weiteren Bindungspartner ein H-Atom, eine ($C_1$-$C_4$)-Alkylkette, Phenyl, Benzyl oder Phenylethyl trägt und der aromatische Rest wie unter R(1)

angegeben substituiert sein kann,
oder

R(1) und R(2) gemeinsam mit dem Stickstoffatom, an das sie gebunden sind, einen 1,2,3,4-Tetrahydrochinolinyl- oder 1,2,3,4-Tetrahydroisochinolinyl-Rest bilden, wobei der aromatische Ring unsubstituiert oder mit bis zu drei Substituenten der Reihe F, Cl, Br, $CF_3$, $CH_3$, $OCH_3$, oder $SCH_3$ substituiert sein kann,

R(3) Phenyl, Biphenylyl, Phenylmethylphenyl, Phenyl-Z-Phenyl, Benzyl, $(C_1$-$C_{10})$-Alkyl ist, wobei die erwähnten aromatischen Ringsysteme unsubstituiert oder mit ein bis drei Substituenten aus der Reihe F, Cl, Br, $OCH_3$, $SCH_3$, $(C_1$-$C_{10})$-Alkyl, $CF_3$, CN, $(C_3$-$C_6)$-Cycloalkyl, $(C_2$-$C_{10})$-Alkenyl substituiert sind und Z für Sauerstoff, Schwefel, Sulfinyl, Sulfonyl steht,
und

R(4) Wasserstoff, $(C_1$-$C_{10})$-Alkyl, $(C_2$-$C_{10})$-Alkenyl, $(C_2$-$C_{10})$-Alkinyl, Phenyl, Benzyl bedeutet, wobei der aromatische Ring wie unter R(3) angegeben unsubstituiert oder ein- bis dreifach substituiert sein kann,
und

Y OH, SH, F, Cl, Br, Z-R(5), -OC(O)R(5), -OCO(O)R(5), -OC(S)R(5) oder NR(6)R(7), worin

R(5) $(C_1$-$C_{15})$-Alkyl, $(C_2$-$C_{15})$-Alkenyl, $(C_3$-$C_8)$-Cycloalkyl, Phenyl, $(C_1$-$C_4)$-Alkyl-phenyl, Hetaryl, $(C_1$-$C_4)$-Alkyl-Hetaryl, Biphenylyl, Phenyl-Z-Phenyl, Phenyl-methyl-Phenyl, Naphthyl bedeutet, wobei die aufgeführten aromatischen und heteroaromatischen Ringsysteme unsubstituiert oder mit ein bis drei Substituenten - gleich oder verschieden - aus der Reihe F, Cl, Br, $CF_3$, CN, $NO_2$, $(C_1$-$C_{10})$-Alkyl, $(C_2$-$C_{10})$-Alkenyl, Z-$(C_1$-$C_{10})$-Alkyl, Z-$(C_2$-$C_{10})$-Alkenyl, $(C_3$-$C_8)$-Cycloalkyl substituiert sind, und

Z Sauerstoff, Schwefel, Sulfinyl oder Sulfonyl ist, und das heteroaromatische Ringsystem ein bis fünf Kohlenstoffatome sowie ein bis drei Heteroatome - gleich oder verschieden - aus der Reihe N, O, S enthält (z.B. seien als Heteroaromaten genannt Thiophen, Pyridin, Furan, Oxazol, Isoxazol, Thiazol, Isothiazol)

R(6) $(C_1$-$C_{10})$-Alkyl (geradkettig oder verzweigt), H bedeutet und

R(7) $(C_1$-$C_{10})$-Alkyl (geradkettig oder verzweigt), Phenyl, Benzyl bedeutet und die Phenyl- oder Benzylgruppe neben Wasserstoff 1-3 F-, Cl-, Br-Atome oder $CF_3$-, $CH_3$- oder $OCH_3$-Gruppen als Substituenten aufweist,
oder wobei

R(6) und R(7) gemeinsam eine Kette aus $(CH_2)_{4-6}$-Einheiten bilden, in welcher eine $CH_2$-Einheit durch ein Stickstoffatom, welches als zusätzlichen Bindungspartner ein Wasserstoffatom, eine Phenyl- oder eine Benzylgruppe trägt, ersetzt sein kann und die Phenyl- oder Benzylgruppe neben Wasserstoff 1-3 F-, Cl-, Br-Atome oder $CF_3$-, $CH_3$- oder $OCH_3$-Gruppen als Substituenten aufweist.

Diese Verbindungen I finden Verwendung in Form der freien Basen und ihrer Salze mit pharmazeutisch akzeptablen Säuren. Die erwähnten Alkyl- und die ungesättigten aliphatischen Reste können geradkettig oder verzweigt sein; ungesättigte aliphatische Reste sind ein- oder mehrfach ungesättigt.

Bevorzugt ist die Verwendung von Verbindungen der Formel I, worin bedeuten:

A CH, N

X -NR(1)R(2), -O-Phenyl, -O-Benzyl (wobei Phenyl und Benzyl unsubstituiert oder mit ein bis drei Substituenten aus der Reihe F, Cl, Br, O-$(C_1$-$C_4)$-Alkyl, $(C_1$-$C_4)$-Alkyl, $CF_3$ substituiert ist), -O-N = CR(3)R(4), worin

R(1) $(C_1$-$C_{10})$-Alkyl, $(C_2$-$C_{10})$-Alkenyl, $(C_3$-$C_8)$-Cycloalkyl, Phenyl, Benzyl, Hetaryl, Methyl-Hetaryl, Biphenylyl, Phenyl-Z-Phenyl, Phenyl-methyl-Phenyl, Naphthyl ist, wobei die aufgeführten aromatischen und heteroaromatischen Ringsysteme unsubstituiert oder mit ein bis drei Substituenten - gleich oder verschieden - aus der Reihe F, Cl, Br, $CF_3$, CN, $(C_1$-$C_{10})$-Alkyl, $(C_2$-$C_{10})$-Alkenyl, Z-$(C_1$-$C_{10})$-Alkyl, Z-$(C_2$-$C_{10})$-Alkenyl substituiert sind, Z für Sauerstoff oder Schwefel steht und das heteroaromatische Ringsystem gleich Thiophen, Pyridin, Furan, Oxazol, Isoxazol, Thiazol oder Isothiazol ist.
und

R(2) Wasserstoff bedeutet oder wie R(1) definiert ist, wobei R(1) und R(2) gleich oder verschieden sind,
oder

R(1) und R(2) gemeinsam eine Kette von $(CH_2)_{4-6}$-Einheiten bilden, in welcher eine $(CH_2)$-Einheit durch ein Sauerstoff-, Schwefel- oder Stickstoffatom ersetzt sein kann und wobei das

| | |
|---|---|
| | zusätzliche N-Atom als weiteren Bindungspartner ein H-Atom, eine Phenyl, Benzyl oder Phenylethyl trägt und der aromatische Rest wie unter R(1) angegeben substituiert sein kann, |
| | oder |
| R(1) und R(2) | gemeinsam mit dem Stickstoffatom, an das sie gebunden sind, einen 1,2,3,4-Tetrahydrochinolinyl- oder 1,2,3,4-Tetrahydroisochinolinyl-Rest bilden, wobei der aromatische Ring unsubstituiert oder mit bis zu zwei Substituenten der Reihe F, Cl, $CF_3$, $CH_3$, $OCH_3$, substituiert sein kann, |
| R(3) | Phenyl, Biphenylyl, Phenylmethylphenyl, Phenyl-Z-Phenyl, Benzyl, ($C_1$-$C_{10}$)-Alkyl ist, wobei die erwähnten aromatischen Ringsysteme unsubstituiert oder mit ein bis drei Substituenten aus der Reihe F, Cl, Br, $OCH_3$, ($C_1$-$C_{10}$)-Alkyl, $CF_3$, ($C_3$-$C_6$)-Cycloalkyl, ($C_2$-$C_{10}$)-Alkenyl substituiert sind und Z für Sauerstoff, oder Schwefel steht, und |
| R(4) | Wasserstoff, ($C_1$-$C_{10}$)-Alkyl, ($C_2$-$C_{10}$)-Alkenyl, Phenyl, Benzyl bedeutet, wobei der aromatische Ring wie unter R(3) angegeben unsubstituiert oder ein- bis dreifach substituiert sein kann, |
| | und |
| Y | OH, F, Cl, Z-R(5), -OC(O)R(5), -OCO(O)R(5),oder NR(6)R(7), worin |
| R(5) | ($C_1$-$C_{10}$)-Alkyl, ($C_2$-$C_{10}$)-Alkenyl, ($C_3$-$C_8$)-Cycloalkyl, Phenyl, Benzyl, Methyl-Hetaryl, Biphenylyl, Phenyl-Z-Phenyl, Phenyl-methyl-Phenyl, Naphthyl bedeutet, wobei die aufgeführten aromatischen und heteroaromatischen Ringsysteme unsubstituiert oder mit ein bis drei Substituenten - gleich oder verschieden - aus der Reihe F, Cl, $CF_3$, CN, ($C_1$-$C_{10}$)-Alkyl, ($C_2$-$C_{10}$)-Alkenyl, Z-($C_1$-$C_{10}$)-Alkyl, Z-($C_2$-$C_{10}$)-Alkenyl, ($C_3$-$C_8$)-Cycloalkyl substituiert sind. |
| R(6) | ($C_1$-$C_{10}$)-Alkyl (geradkettig oder verzweigt, bedeutet und |
| R(7) | ($C_1$-$C_{10}$)-Alkyl (geradkettig oder verzweigt), Phenyl, Benzyl bedeutet und die Phenyl- oder Benzylgruppe neben Wasserstoff 1-3 F-, Cl-Atome oder $CF_3$-, $CH_3$- oder $OCH_3$-Gruppen als Substituenten aufweist, |
| | oder wobei |
| R(6) und R(7) | gemeinsam eine Kette aus $(CH_2)_{4-6}$-Einheiten bilden, in welcher eine $CH_2$-Einheit durch ein Stickstoffatom, welches als zusätzlichen Bindungspartner ein Wasserstoffatom eine Phenyl- oder eine Benzylgruppe trägt, ersetzt sein kann und die Phenyl- oder Benzylgruppe neben Wasserstoff 1-4 F- oder Cl-Atome oder $CF_3$-, $CH_3$- oder $OCH_3$-Gruppen als Substituenten aufweist |
| | und |
| Z | Sauerstoff oder Schwefel ist, und das heteroaromatische Ringsystem ein Heterocyclus aus der Reihe Thiophen, Pyridin, Furan, Oxazol, Isoxazol, Thiazol, Isothiazol ist. |

Besonders bevorzugt ist die Verwendung von Verbindungen der Formel I, worin bedeuten:

| | |
|---|---|
| A | CH, N |
| X | -NR(1)R(2), -O-Phenyl, -O-Benzyl, wobei Phenyl und Benzyl unsubstituiert oder mit ein bis zwei Substituenten aus der Reihe F, Cl, O-($C_1$-$C_4$)-Alkyl, ($C_1$-$C_4$)-Alkyl, substituiert ist), -O-N = CR(3)R(4), worin |
| R(1) | ($C_1$-$C_{10}$)-Alkyl, ($C_2$-$C_{10}$)-Alkenyl, ($C_3$-$C_6$)-Cycloalkyl, Phenyl, Benzyl, Biphenylyl, Phenyl-Z-Phenyl, Phenyl-methyl-Phenyl ist, wobei die aufgeführten aromatischen Ringsysteme unsubstituiert oder mit ein bis zwei Substituenten - gleich oder verschieden - aus der Reihe F, Cl, $CF_3$, ($C_1$-$C_{10}$)-Alkyl, ($C_2$-$C_{10}$)-Alkenyl, Z-($C_1$-$C_{10}$)-Alkyl, Z-($C_2$-$C_{10}$)-Alkenyl, substituiert sind und Z für Sauerstoff oder Schwefel steht |
| | und |
| R(2) | Wasserstoff bedeutet oder wie R(1) definiert ist, wobei R(1) und R(2) gleich oder verschieden sind, |
| | oder |
| R(1) und R(2) | gemeinsam eine Kette von $(CH_2)_{4-5}$-Einheiten bilden, in welcher eine $(CH_2)$-Einheit durch ein Stickstoffatom ersetzt sein kann und wobei das zusätzliche N-Atom als weiteren Bindungspartner ein Phenyl oder Benzyl trägt und der aromatische Rest wie unter R(1) angegeben substituiert sein kann, |
| | oder |
| R(1) und R(2) | gemeinsam mit dem Stickstoffatom, an das sie gebunden sind, einen 1,2,3,4-Tetrahydrochinolinyl- oder 1,2,3,4-Tetrahydroisochinolinyl-Rest bilden, wobei der aromatische Ring unsubstituiert oder mit bis zu zwei Substituenten der Reihe F, Cl, $CF_3$, |

$CH_3$, $OCH_3$, substituiert sein kann,

R(3) Phenyl, Biphenylyl, Phenylmethylphenyl, Phenyl-Z-Phenyl, Benzyl, $(C_1-C_{10})$-Alkyl ist, wobei die erwähnten aromatischen Ringsysteme unsubstituiert oder mit ein bis zwei Substituenten aus der Reihe F, Cl, $OCH_3$, $(C_1-C_{10})$-Alkyl, $CF_3$, $(C_3-C_6)$-Cycloalkyl, $(C_2-C_8)$-Alkenyl substituiert sind und Z für Sauerstoff oder Schwefel steht,

und

R(4) Wasserstoff, $(C_1-C_{10})$-Alkyl, $(C_2-C_{10})$-Alkenyl, Phenyl, Benzyl bedeutet, wobei der aromatische Ring wie unter R(3) angegeben unsubstituiert oder ein- bis zweifach substituiert sein kann,

und

Y OH, F, Cl, Z-R(5), -OC(O)R(5), -OCO(O)R(5), oder NR(6)R(7) worin

R(5) $(C_1-C_{10})$-Alkyl, $(C_2-C_{10})$-Alkenyl, Phenyl, Benzyl, Biphenylyl, Phenyl-Z-Phenyl, Phenylmethyl-Phenyl, bedeutet, wobei die aufgeführten aromatischen Ringsysteme unsubstituiert oder mit ein bis zwei Substituenten - gleich oder verschieden - aus der Reihe F, Cl, $CF_3$, $(C_1-C_{10})$-Alkyl, $(C_2-C_{10})$-Alkenyl, Z-$(C_1-C_{10})$-Alkyl, Z-$(C_2-C_{10})$-Alkenyl, $(C_3-C_8)$-Cycloalkyl substituiert sind,

R(6) $(C_1-C_{10})$-Alkyl (geradkettig oder verzweigt, bedeutet und

R(7) $(C_1-C_{10})$-Alkyl (geradkettig oder verzweigt), Phenyl, Benzyl bedeutet und die Phenyl- oder Benzylgruppe neben Wasserstoff 1-3 F- oder Cl-Atome oder $CF_3$-, $CH_3$- oder $OCH_3$-Gruppen als Substituenten aufweist,

oder wobei

R(6) und R(7) gemeinsam eine Kette aus $(CH_2)_{4-5}$-Einheiten bilden, in welcher eine $CH_2$-Einheit durch ein Stickstoffatom welches als zusätzlichen Bindungspartner ein Wasserstoffatom eine Phenyl- oder eine Benzylgruppe trägt, ersetzt sein kann und die Phenyl- oder Benzylgruppe neben Wasserstoff 1-2 F- oder Cl-Atome oder $CF_3$-, $CH_3$- oder $OCH_3$-Gruppen als Substituenten aufweist

und

Z Sauerstoff oder Schwefel ist.

Beispiele pharmazeutisch akzeptabler salzbildender Säuren sind organische Säuren wie Salzsäure, Bromwasserstoffsäure Jodwasserstoffsäure, Schwefelsäure, Phosphorsäure oder Salpetersäure oder organische Säuren wie Malonsäure-, Oxalsäure, Gluconsäure, Camphersulfonsäure, Benzolsulfonsäure, Essigsäure, Propionsäure, p-Toluolsulfonsäure oder Salicylsäure.

Die Verbindungen I weisen mindestens ein asymmetrisches C-Atom auf und können daher als Enantiomere und Diasteromere auftreten. Die Erfindung umfaßt sowohl die Verwendung der reinen Isomeren als auch von deren Gemischen. Gemische von Diastereomeren können nach gebräuchlichen Methoden, z.B. durch selektive Kristallisation aus geeigneten Lösungsmitteln oder durch Chromatographie an Kieselgel oder Aluminiumoxid in die Komponenten aufgetrennt werden.

Racemate können nach üblichen Methoden in die Enantiomeren aufgetrennt werden, z.B. durch Salzbildung mit einer optisch aktiven Säure, Trennung der diastereomeren Salze und Freisetzung der reinen Enantiomeren mittels einer Base. Darüber hinaus lassen sich die Enantiomeren auch mittels Chromatographie an chiralen Phasen oder auf enzymatischem Wege trennen.

Die Verbindungen der Formel I, ihre Säureadditionssalze und ihre physiologisch hydrolysierbaren Derivate sind wertvolle Arzneimittel. Sie wirken insbesondere antimikrobiell und eignen sich besonders zur Vorbeugung gegen und Behandlung von Pilzinfektionen beim Menschen und bei verschiedenen Säugetierarten.

Die Verbindungen sind in vitro sehr gut wirksam gegen Hautpilze, wie z.B. Trichophyton mentagrophytes, Microsporum canis, Epidermophyton floccosum; gegen Schimmelpilze, wie z.B. Aspergillus niger oder gegen Hefen, wie z.B. Candida albicans, C. tropicalis, Torulopsis glabrata und Trichosporon cutaneum oder gegen Protozoen wie Trichomonas vaginalis oder T. fetus, oder auch gegen einzelne Bakterienspezies.

Auch in vivo, z.B. bei der experimentellen Nierencandidose der Maus, besitzen die Verbindungen nach oraler oder parenteraler Anwendung einen sehr guten systemischen Effekt, z.B. gegen Candida albicans. Hierbei wird von der Hefe Candida albicans insbesondere das Exoenzymsystem dergestalt beeinflußt, daß die Pathogenität der Erreger deutlich absinkt. Ebenso besteht ein sehr guter Effekt gegen verschiedene Erreger der Hautmykosen (z.B. Trichophyton mentagrophytes) am Meerschweinchen nach oraler, parenteraler oder lokaler Anwendung.

Als Indikationsgebiete in der Humanmedizin können beispielsweise genannt werden:
Dermatomykosen und Systemmykosen durch Trichophyton mentagrophytes und andere Trichophytonarten, Mikrosporumarten, Epidermophyton floccosum, und biphasische Pilze sowie Schimmelpilze hervorgerufen. Insbesondere werden tiefe Mykosen, die durch Candida albicans hervorgerufen werden, günstig beeinflußt, da hierbei ein Eindringen der Pilze in die Wirtszelle verhindert bzw. erschwert wird.

Als Indikationsgebiete in der Tiermedizin können beispielsweise aufgeführt werden:
Alle Dermatomykosen und Systemmykosen, insbesondere solche, die durch die oben genannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere Wirkstoffe enthalten oder die aus einem oder mehreren erfindungsgemäß verwendeten Wirkstoffen bestehen sowie Verfahren zur Herstellung dieser Zubereitungen.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Ein Hemmstoff für die unterschiedlichen Phospholipasen von Candida albicans muß im Patienten überall dort in hinreichenden Konzentrationen vorliegen, wo der Pilz die Parenchyme besiedeln kann. Dieser Umstand setzt voraus, daß die entsprechenden Substanzen in einer Konzentration verabreicht werden müssen, die sich zuvor in Tierexperimenten als wirksam erwiesen hat.

Bei den schweren Krankheitsbildern der tiefen Candidose befinden sich die Patienten meist in einem sehr schlechten Allgemeinzustand. Hohes Fieber und weitere Erkrankungen sind häufig anzutreffen. Bei den Dosierungsvorgaben muß zwischen der prophylaktischen Gabe und der Therapie im nachgewiesenen Infektionsfall unterschieden werden. Bei der Prophylaxe kann von einem besseren Allgemeinzustand der Patienten ausgegangen werden, der oft eine orale Verabreichung ermöglicht. Hierbei können Tabletten, Lösungen, Gele oder Trockensaft zum Einsatz kommen. Bei den Formen mit nachgewiesenen tiefen Candidosen muß oft davon ausgegangen werden, daß eine geregelte orale Aufnahme der Wirkstoffe nicht immer gewährleistet ist. Hierfür kommen dann parenterale Anwendungsformen in Frage. Im Ausnahmefall kann auch an eine subcutane Verabreichung gedacht werden.

Als Kandidaten für eine Prophylaxe kommen in erster Linie immunkomprimierte Patienten in Frage, die durch entsprechende medikamentöse Belastung oder durch körpereigene Immunprobleme in dieser Situation sind. Dies sind insbesondere Transplantationspatienten, Zuckerkranke und/oder adipöse Patienten, AIDS-Patienten, unter Chemotherapie stehende Patienten, Langzeitbeatmete usw..

Die Verbindungen zeigen Hemmungen der Phospholipase von Candida albicans, die weit unter der in vitro festgestellten minimalen inhibitorischen Konzentrationen der Wirkstoffe gegenüber Candida albicans liegen. Daher kann die Dosierung im Regelfall unter derjenigen liegen, die für eine reine antimykotische Therapie nötig wäre.

Die Wirkung im Patienten beruht darauf, daß die Wirkstoffe bei den Candida Zellen, die sich in der Nähe der Parenchyme befinden, zwei unterschiedliche Effekte auslöst. Zum einen wird die Adhäsion der Hefezellen an die Körperzellen verhindert und zum anderen wird Candida albicans daran gehindert, die Körperzellen mit Keimschläuchen zu penetrieren. Durch dieses duale Wirkungskonzept kann die Hefe ihre Pathogenität nicht zur vollen Ausprägung bringen. Allerdings muß erwähnt werden, das Candida albicans außer den Phospholipasen noch weitere Pathomechanismen wie z.B. die Protease besitzt. Die Anheftung an körpereigene Zellen ist jedoch der primäre Schritt zur Penetration. Da diese Anheftung durch Phospholipasehemmer verhindert wird, können die anderen Pathomechanismen nicht zur Geltung kommen.

Als Darreichungsformen kommen beispielsweise Tabletten, Dragees, Kapseln, Pillen, wäßrige Lösungen, Suspensionen und Emulsionen, gegebenenfalls sterile injizierbare Lösungen, nichtwäßrige Emulsionen, Suspensionen und Lösungen, Salben, Cremes, Pasten, Lotions, Sprays etc. in Betracht.

Die prophylaktisch und therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäß verwendeten Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des Wirkstoffs oder der Wirkstoffe mit dem Trägerstoff oder den Trägerstoffen.

Zur vorliegenden Erfindung gehört die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben angeführten Erkrankungen.

Die Wirkstoffe oder die pharmazeutischen Zubereitungen können lokal, oral, parenteral, intraperitoneal und/oder rectal appliziert werden.

Im allgemeinen hat es sich sowohl in der Human- als auch in der Veterinärmedizin als vorteilhaft erwiesen, den oder die erfindungsgemäß verwendeten Wirkstoffe in Gesamtmengen von mindestens etwa 0,05, vorzugsweise 0,1 , insbesondere 0,5 mg/kg Körpergewicht bis höchstens etwa 200, vorzugsweise bis 100, insbesondere bis 10 mg/kg Körpergewicht je 24 Stunden, gegebenenfalls in Form mehrerer Einzelgaben zur Erzielung der gewünschten Ergebnisse zu verabreichen. Die Gesamtmenge wird in 1 bis 8, vorzugsweise in 1 bis 3 Einzeldosen, bei tiefen Mykosen jedoch über wesentlich längere Zeiträume (bis zu 6 Wochen) verabreicht.

Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Als Beispiel für die Hemmung der pathogenen Phase dimorpher Hefezellen werden Ergebnisse eines in-vitro-Enzymtests angeführt, in welchen die prozentuale Hemmung freigesetzter Exoenzyme, insbesondere freigesetzter Lysophospholipase (Phospholipase B), als Maß für die Wirksamkeit bestimmt wird.

Zur Feststellung der Enzymhemmung wurde eine Suspension von Candida albicans Blastokonidien (Stamm 200/175), wobei die Keimdichte photometrisch auf eine Extinktion von 0,5 (500 nm) eingestellt war, mit Präparatelösung bzw. zur Kontrolle mit Lösungsmittellösung vermischt und zwar

a) 100 µl Präparatelösung (Suspension)
+ 900 µl Keimsuspension

b) 100 µl Lösungsmittel
+ 900 µl Keimsuspension

Jeweils 5 µl der 30 min bei 21 °C inkubierten Blastokonidiensuspension wurden auf eine Agarplatte (Sabourand Agar mit Zusatz von 8 % Eigelb, 1 M NaCl, 5 mM $CaCl_2$) aufgetropft.

Die so beimpfte Platte wurde 3 Tage bei 37 °C bebrütet.

Die Auswertung erfolgte derart, daß

1. der Durchmesser (mm) der Candida albicans-Kolonie (behandelt und unbehandelt) sowie
2. der Gesamtdurchmesser von Kolonie und Trübungshof, welcher durch Exoenzyme verursacht wurde (behandelt und unbehandelt) bestimmt wurde.

Aus der Bestimmung des Quotienten von Präparate- und Kontrollgruppe ergab sich ein Wert, der als Maß für die Enzymaktivität anzusehen war.

Wie aus Tabelle 1 ersichtlich, hemmen die erfindungsgemäß verwendeten Verbindungen die freigesetzten Exoenzyme wesentlich stärker als handelsübliche Betablocker (Propranolol).

Außerdem wird deutlich, daß zur Erreichung einer ausgeprägten Hemmwirkung der Phospholipase-Aktivität Dosen ausreichen, die deutlich unterhalb derjenigen liegen, die zur Hemmung des Pilzwachstums in vitro nötig sind und welche bisher für eine reine antimykotische Therapie als unbedingt erforderlich erachtet wurden (sihe MHK-Werte von Candida albicans einiger ausgewählter Derivate).

Der Stand der Technik wird durch die erfindungsgemäß verwendeten Verbindungen I überraschenderweise deutlich übertroffen.

Tabelle 1

Prozentuale Hemmung der freigesetzten Exoenzyme von Candida albicas (in vitro), MHK-Werte einiger Derivate bezüglich Candida albicans

| | Konzentration µg/ml | % Hemmung der Phospholipase | MHK von Candida albicans µg/ml |
|---|---|---|---|
| 1.1 | | | |
| 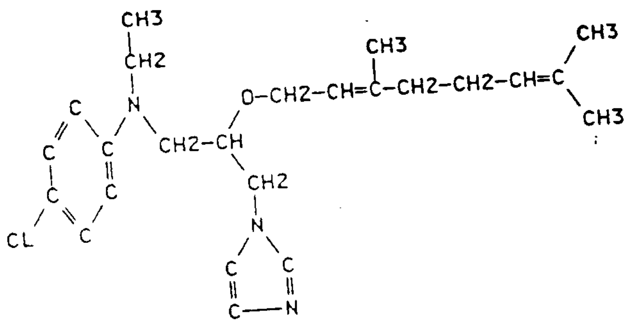 | 10 | 100 | >125 |
| | 5 | 60 | |
| | 1 | 15 | |
| 1.2 | | | |
| 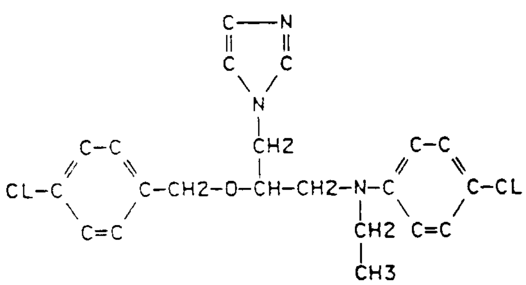 | 50 | 90 | |
| 1.3 | | | |
| 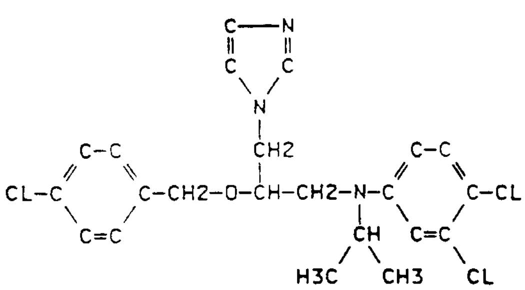 | 50 | 100 | 125 |
| | 10 | 25 | |

| | Konzentration µg/ml | % Hemmung der Phospholipase | MHK von Candida albicans µg/ml |
|---|---|---|---|
| 1.4 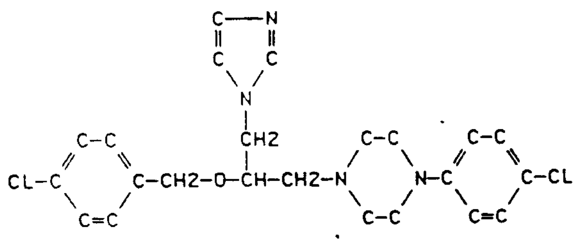 | 50 | 100 | |
| 1.5 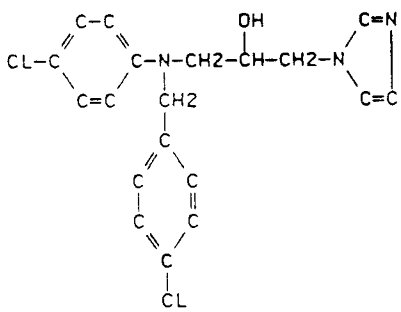 | 50 | 100 | |
| 1.6 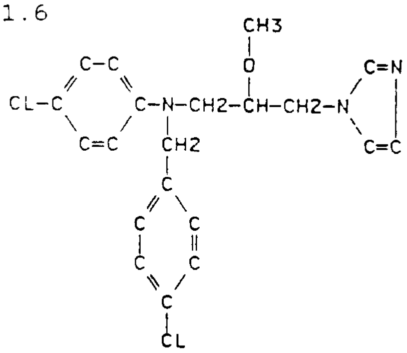 | 50 | 100 | |
| 1.7 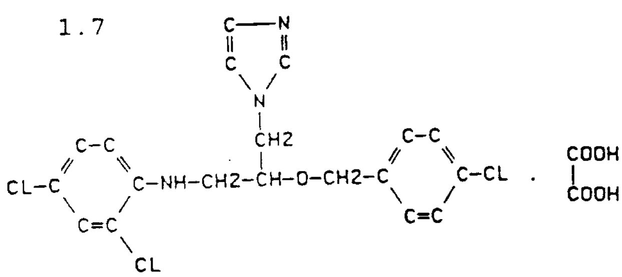 | 50 | 42 | |

| | Konzen-tration µg/ml | % Hemmung der Phos-pholipase | MHK von Candida albicans µg/ml |
|---|---|---|---|
| 1.8 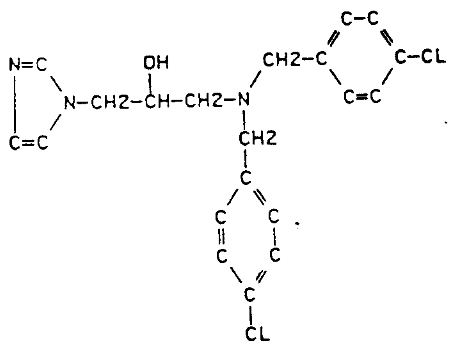 | 50 | 100 | |
| 1.9 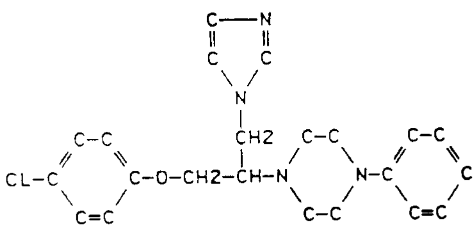 | 50 | 100 | |
| 1.10 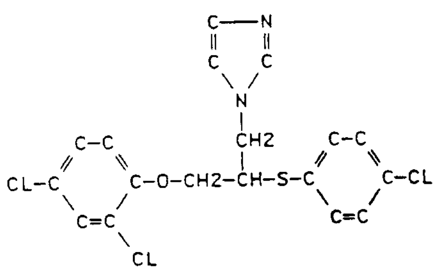 | 50 | 50 | |
| 1.11 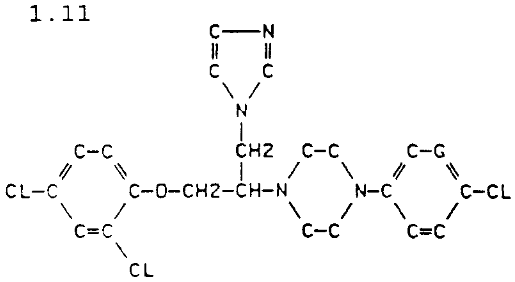 | 50 | 100 | |

| Verbindung | | Konzentration µg/ml | % Hemmung der Phospholipase | MHK von Candida albicans µg/ml |
|---|---|---|---|---|
| 1.12 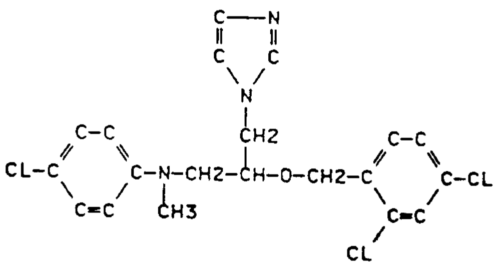 | 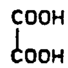 | 50 | 100 | |
| 1.13 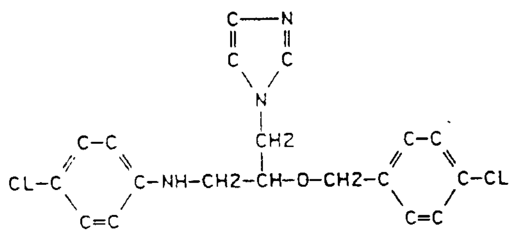 | 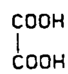 | 100<br>50 | 33<br>26 | |
| 1.14 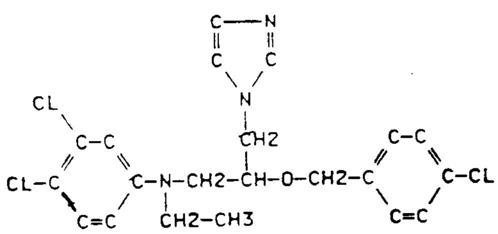 | 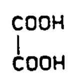 | 100<br>50 | 100<br>30 | |
| 1.15 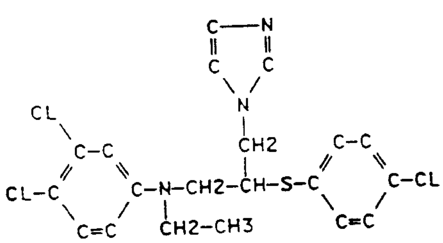 | 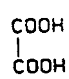 | 50 | 100 | 125 |

| | Konzentration µg/ml | % Hemmung der Phospholipase | MHK von Candida albicans µg/ml |
|---|---|---|---|
| 1.16 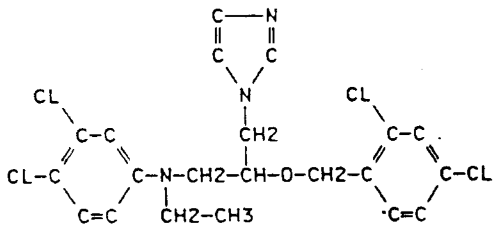 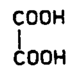 | 50 | 100 | 125 |
| 1.17 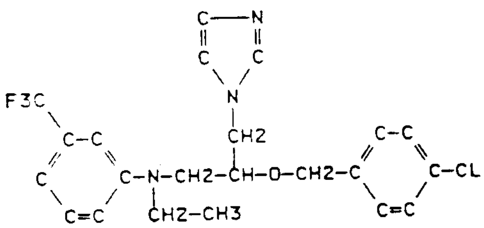 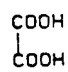 | 100<br>50 | 38<br>12 | |
| 1.18 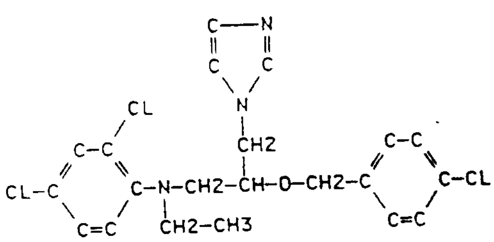 | 50 | 60 | |
| 1.19 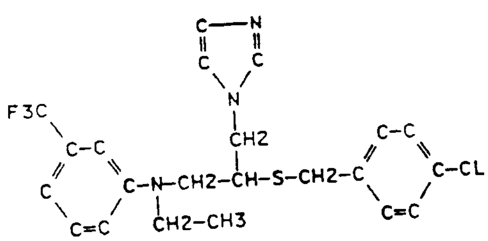 | 50 | 71,4 | 125 |

| | Konzentration µg/ml | % Hemmung der Phospholipase | MHK von Candida albicans µg/ml |
|---|---|---|---|
| 1.20 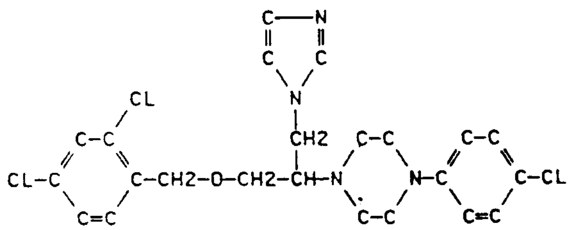 | 50 | 100 | |
| 1.21 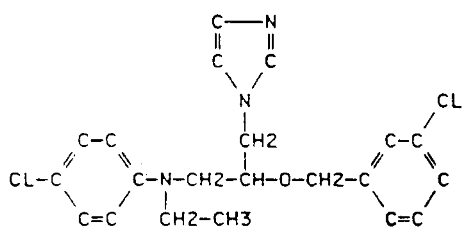 | 50<br>10 | 50<br>12,7 | 125 |
| 1.22 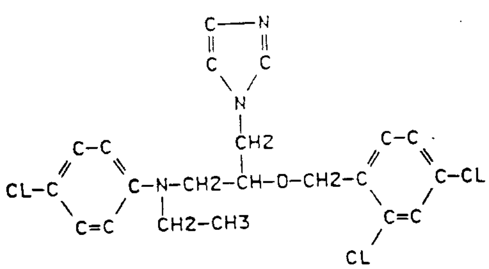 . 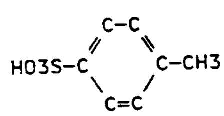 | 50<br>10 | 100<br>23,1 | 125 |
| 1.23 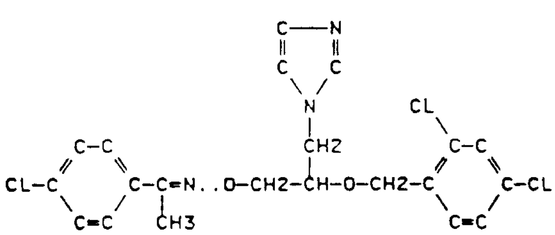 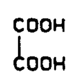 | 50<br>10 | 100<br>35,7 | |

| | Konzentration µg/ml | % Hemmung der Phospholipase | MHK von Candida albicans µg/ml |
|---|---|---|---|
| 1.24 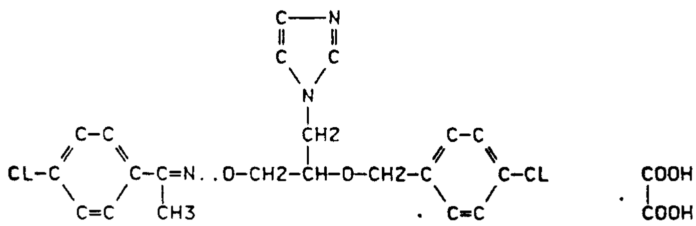 | 50 | 50 | |
| | 10 | 24,3 | |
| 1.25 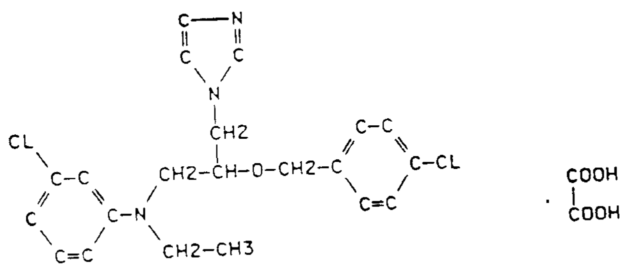 | 50 | 60 | |
| | 10 | 27,3 | |
| 1.26 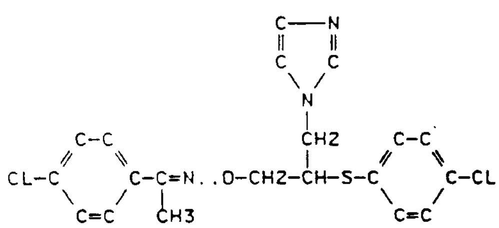 | 50 | 90 | |
| | 10 | 50 | |
| 1.27 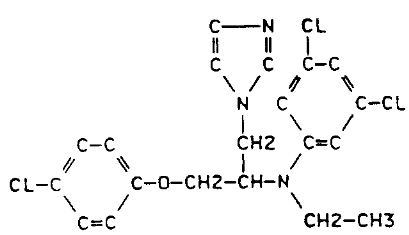 | 50 | 100 | |

| | Konzentration µg/ml | % Hemmung der Phospholipase | MHK von Candida albicans µg/ml |
|---|---|---|---|
| 1.28 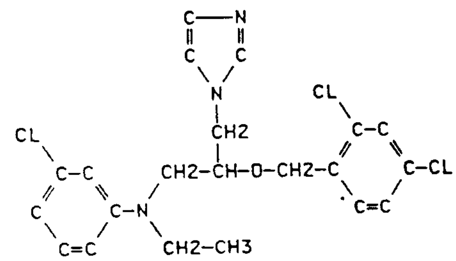 | 100<br>50 | 100<br>61,5 | 125 |
| 1.29 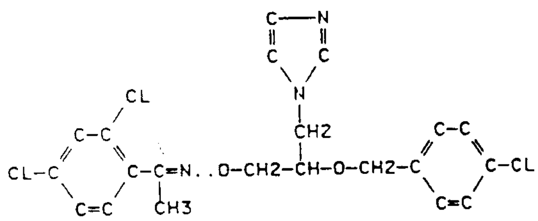 | 50<br>10 | 100<br>20,6 | |
| 1.30 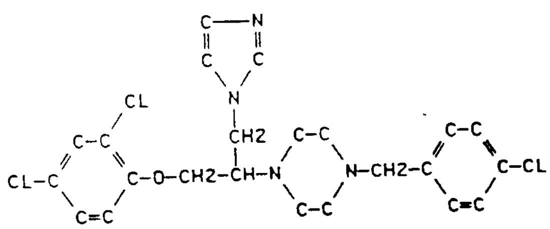 | 50 | 100 | |
| 1.31 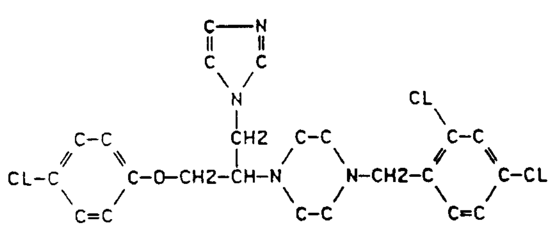 | 100<br>50 | 90<br>38,6 | |

| | Konzentration µg/ml | % Hemmung der Phospholipase | MHK von Candida albicans µg/ml |
|---|---|---|---|
| 1.32 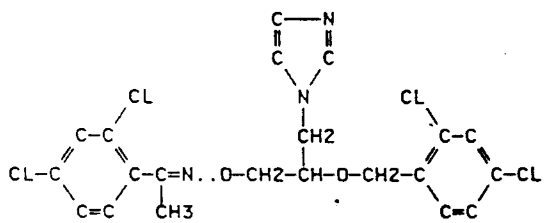 | 50<br>10 | 100<br>18,2 | 125 |
| 1.33 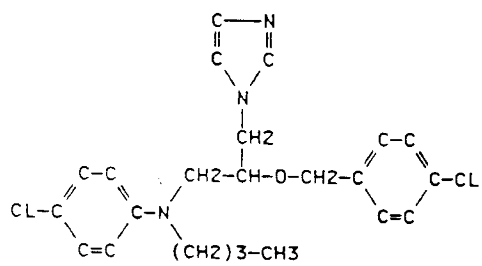 | 50<br>10 | 80<br>24 | 125 |
| 1.34 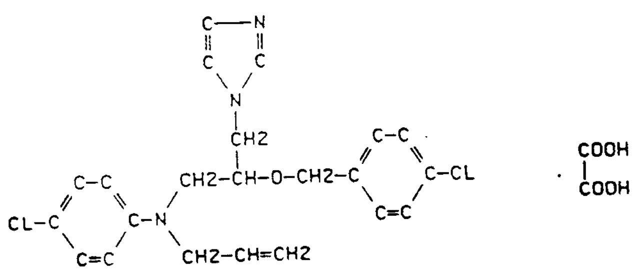 | 50<br>10 | 100<br>10,7 | |
| 1.35 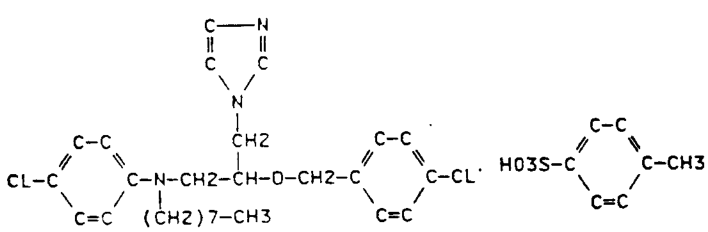 | 50<br>10 | 100<br>45 | >125 |

| | Konzen-tration µg/ml | % Hemmung der Phos-pholipase | MHK von Candida albicans µg/ml |
|---|---|---|---|
| 1.36 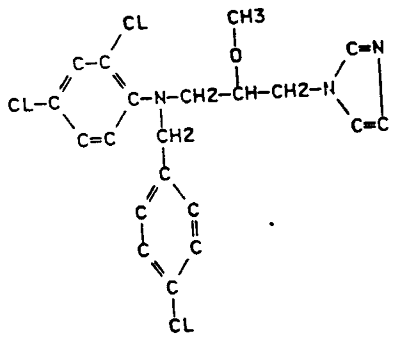 | 50 | 100 | |
| 1.37 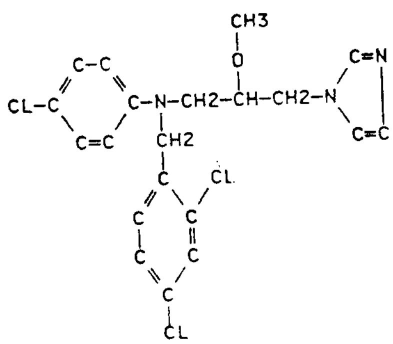 . 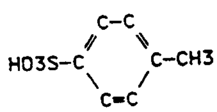 | 50<br>10 | 100<br>16,7 | |
| 1.38 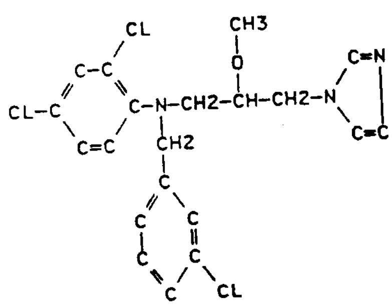 | 50<br>10 | 100<br>16,7 | |
| 1.39 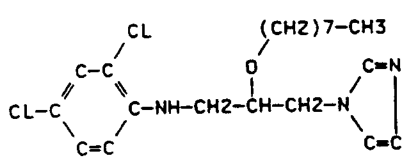 | 50<br>10 | 100<br>85,3 | |

| | Konzentration µg/ml | % Hemmung der Phospholipase | MHK von Candida albicans µg/ml |
|---|---|---|---|
| 1.40 | | | |
| 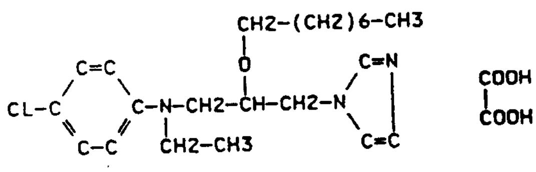 | 50<br>10 | 100<br>75,0 | 125 |
| 1.41 | | | |
| 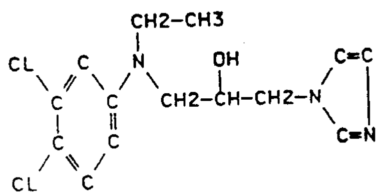 | 50<br>10 | 100<br>33,3 | |
| 1.42 | | | |
| 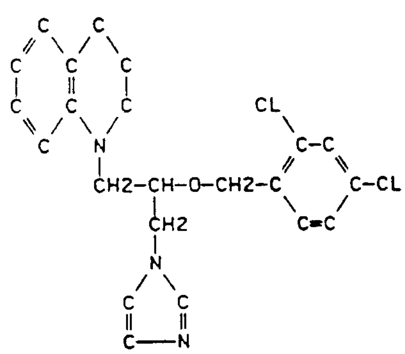 | 50 | 100 | |
| 1.43 | | | |
| 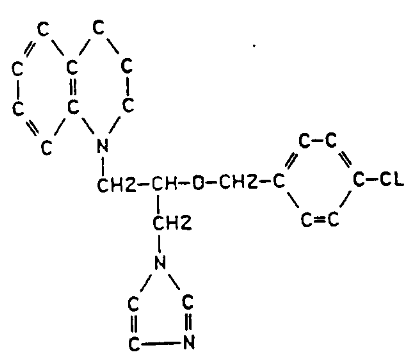 | 50<br>10 | 100<br>50 | |

## Patentansprüche

1. Verwendung von 2,3-disubstituierten 1-Azolylpropanen I

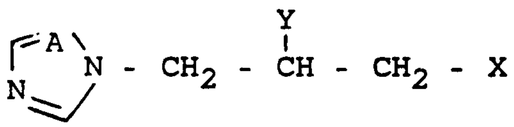 I,

in denen bedeuten

A CH, N

X -NR(1)R(2), -O-Phenyl, -O-Benzyl, S-Phenyl, S-Benzyl, (wobei Phenyl und Benzyl unsubstituiert oder mit ein bis drei Substituenten aus der Reihe F, Cl, Br, O-($C_1$-$C_4$)-Alkyl, ($C_1$-$C_4$)-Alkyl, $CF_3$ substituiert ist), -O-N = CR(3)R(4), worin

R(1) ($C_1$-$C_{15}$)-Alkyl, ($C_2$-$C_{15}$)-Alkenyl, ($C_2$-$C_{15}$)-Alkinyl, ($C_3$-$C_{15}$)-Alken-in-yl, ($C_3$-$C_8$)-Cycloalkyl, Phenyl, ($C_1$-$C_4$)-Alkyl-Phenyl, Hetaryl, ($C_1$-$C_4$)-Alkyl-Hetaryl, Biphenylyl, Phenyl-Z-Phenyl, Phenyl-methyl-Phenyl, Naphthyl ist, wobei die aufgeführten aromatischen und heteroaromatischen Ringsysteme unsubstituiert oder mit ein bis drei Substituenten - gleich oder verschieden - aus der Reihe F, Cl, Br, $CF_3$, CN, $NO_2$, ($C_1$-$C_{10}$)-Alkyl, ($C_2$-$C_{10}$)-Alkenyl, Z-($C_1$-$C_{10}$)-Alkyl, Z-($C_2$-$C_{10}$)-Alkenyl, Z-($C_3$-$C_8$)-Cycloalkyl substituiert sind, Z für Sauerstoff, Schwefel, Sulfinyl, Sulfonyl steht und das heteroaromatische Ringsystem ein bis fünf Kohlenstoffatome sowie ein bis drei Heteroatome - gleich oder verschieden - aus der Reihe N, O, S enthält (z.B. seien als Heteroaromaten genannt Thiophen, Pyridin, Furan, Oxazol, Isoxazol, Thiazol, Isthiazol)

und

R(2) Wasserstoff bedeutet oder wie R(1) definiert ist, wobei R(1) und R(2) gleich oder verschieden sind,

oder

R(1) und R(2) gemeinsam eine Kette von $(CH_2)_{4-6}$-Einheiten bilden, in welcher eine ($CH_2$)-Einheit durch ein Sauerstoff-, Schwefel- oder Stickstoffatom ersetzt sein kann und wobei das zusätzliche N-Atom als weiteren Bindungspartner ein H-Atom, eine ($C_1$-$C_4$)-Alkylkette, Phenyl, Benzyl oder Phenylethyl trägt und der aromatische Rest wie unter R(1) angegeben substituiert sein kann,

oder

R(1) und R(2) gemeinsam mit dem Stickstoffatom, an das sie gebunden sind, einen 1,2,3,4-Tetrahydrochinolinyl- oder 1,2,3,4-Tetrahydroisochinolinyl-Rest bilden, wobei der aromatische Ring unsubstituiert oder mit bis zu drei Substituenten der Reihe F, Cl, Br, $CF_3$, $CH_3$, $OCH_3$, oder $SCH_3$ substituiert sein kann,

R(3) Phenyl, Biphenylyl, Phenylmethylphenyl, Phenyl-Z-Phenyl, Benzyl, ($C_1$-$C_{10}$)-Alkyl ist, wobei die erwähnten aromatischen Ringsysteme unsubstituiert oder mit ein bis drei Substituenten aus der Reihe F, Cl, Br, $OCH_3$, $SCH_3$, ($C_1$-$C_{10}$)-Alkyl, $CF_3$, CN, ($C_3$-$C_6$)-Cycloalkyl, ($C_2$-$C_{10}$)-Alkenyl substituiert sind und Z für Sauerstoff, Schwefel, Sulfinyl, Sulfonyl steht,

und

R(4) Wasserstoff, ($C_1$-$C_{10}$)-Alkyl, ($C_2$-$C_{10}$)-Alkenyl, ($C_2$-$C_{10}$)-Alkinyl, Phenyl, Benzyl bedeutet, wobei der aromatische Ring wie unter R(3) angegeben unsubstituiert oder ein bis dreifach substituiert sein kann,

und

Y OH, SH, F, Cl, Br, Z(1)-R(5), -OC(O)R(5), -OCO(O)R(5), -OC(S)R(5) oder NR(6)R-(7), worin

R(5) ($C_1$-$C_{15}$)-Alkyl, ($C_2$-$C_{15}$)-Alkenyl, ($C_3$-$C_8$)-Cycloalkyl, Phenyl, ($C_1$-$C_4$)-Alkyl-phenyl, Hetaryl, ($C_1$-$C_4$)-Alkyl-Hetaryl, Biphenylyl, Phenyl-Z-Phenyl, Phenyl-methyl-Phenyl, Naphthyl bedeutet, wobei die aufgeführten aromatischen und heteroaromatischen Ringsysteme unsubstituiert oder mit ein bis drei Substituenten - gleich oder verschieden - aus der Reihe F, Cl, Br, $CF_3$, CN, $NO_2$, ($C_1$-$C_{10}$)-Alkyl, ($C_2$-$C_{10}$)-Alkenyl, Z-($C_1$-$C_{10}$)-Alkyl, Z-($C_2$-$C_{10}$)-Alkenyl, ($C_3$-$C_8$)-Cycloalkyl substituiert sind,

und

Z Sauerstoff, Schwefel, Sulfinyl oder Sulfonyl ist, und das heteroaromatische Ringsystem ein bis fünf Kohlenstoffatome sowie ein bis drei Heteroatome - gleich oder

verschieden - aus der Reihe N, O, S enthält (z.B. seien als Heteroaromaten genannt Thiophen, Pyridin, Furan, Oxazol, Isoxazol, Thiazol, Isothiazol)

R(6) $(C_1-C_{10})$-Alkyl (geradkettig oder verzweigt), H bedeutet und

R(7) $(C_1-C_{10})$-Alkyl (geradkettig oder verzweigt), Phenyl, Benzyl bedeutet und die Phenyl- oder Benzylgruppe neben Wasserstoff 1-3 F-, Cl-, Br-Atome oder $CF_3$-, $CH_3$- oder $OCH_3$-Gruppen als Substituenten aufweist,

oder wobei

R(6) und R(7) gemeinsam eine Kette aus $(CH_2)_{4-6}$-Einheiten bilden, in welcher eine $CH_2$-Einheit durch ein Stickstoffatom, welches als zusätzlichen Bindungspartner ein Wasserstoffatom, eine Phenyl- oder eine Benzylgruppe trägt, ersetzt sein kann und die Phenyl- oder Benzylgruppe neben Wasserstoff 1-3 F-, Cl-, Br-Atome oder $CF_3$-, $CH_3$- oder $OCH_3$-Gruppen als Substituenten aufweist

bedeutet, zum Herstellen eines Medikaments zur Prophylaxe und zur Behandlung von systemischen Mykosen.

**2.** Verwendung von Verbindungen I nach Anspruch 1, in denen bedeuten:

A CH, N

X -NR(1)R(2), -O-Phenyl, -O-Benzyl (wobei Phenyl und Benzyl unsubstituiert oder mit ein bis drei Substituenten aus der Reihe F, Cl, Br, O-$(C_1-C_4)$-Alkyl, $(C_1-C_4)$-Alkyl, $CF_3$ substituiert ist), -O-N=CR(3)R(4), worin

R(1) $(C_1-C_{10})$-Alkyl, $(C_2-C_{10})$-Alkenyl, $(C_3-C_8)$-Cycloalkyl, Phenyl, Benzyl, Hetaryl, Methyl-Hetaryl, Biphenylyl, Phenyl-Z-Phenyl, Phenyl-methyl-Phenyl, Naphthyl ist, wobei die aufgeführten aromatischen und heteroaromatischen Ringsysteme unsubstituiert oder mit ein bis drei Substituenten - gleich oder verschieden - aus der Reihe F, Cl, Br, $CF_3$, CN, $(C_1-C_{10})$-Alkyl, $(C_2-C_{10})$-Alkenyl, Z-$(C_1-C_{10})$-Alkyl, Z-$(C_2-C_{10})$-Alkenyl substituiert sind, Z für Sauerstoff oder Schwefel steht und das heteroaromatische Ringsystem gleich Thiophen, Pyridin, Furan, Oxazol, Isoxazol, Thiazol oder Isothiazol ist,

und

R(2) Wasserstoff bedeutet oder wie R(1) definiert ist, wobei R(1) und R(2) gleich oder verschieden sind,

oder

R(1) und R(2) gemeinsam eine Kette von $(CH_2)_{4-6}$-Einheiten bilden, in welcher eine $(CH_2)$-Einheit durch ein Sauerstoff-, Schwefel- oder Stickstoffatom ersetzt sein kann und wobei das zusätzliche N-Atom als weiteren Bindungspartner ein H-Atom, eine Phenyl, Benzyl oder Phenylethyl trägt und der aromatische Rest wie unter R(1) angegeben substituiert sein kann,

oder

R(1) und R(2) gemeinsam mit dem Stickstoffatom, an das sie gebunden sind, einen 1,2,3,4-Tetrahydrochinolinyl- oder 1,2,3,4-Tetrahydroisochinolinyl-Rest bilden, wobei der aromatische Ring unsubstituiert oder mit bis zu zwei Substituenten der Reihe F, Cl, $CF_3$, $CH_3$, $OCH_3$, substituiert sein kann,

R(3) Phenyl, Biphenylyl, Phenylmethylphenyl, Phenyl-Z-Phenyl, Benzyl, $(C_1-C_{10})$-Alkyl ist, wobei die erwähnten aromatischen Ringsysteme unsubstituiert oder mit ein bis drei Substituenten aus der Reihe F, Cl, Br, $OCH_3$, $(C_1-C_{10})$-Alkyl, $CF_3$, $(C_3-C_6)$-Cycloalkyl, $(C_2-C_{10})$-Alkenyl substituiert sind und Z für Sauerstoff, oder Schwefel steht,

und

R(4) Wasserstoff, $(C_1-C_{10})$-Alkyl, $(C_2-C_{10})$-Alkenyl, Phenyl, Benzyl bedeutet, wobei der aromatische Ring wie unter R(3) angegeben unsubstituiert oder ein bis dreifach substituiert sein kann,

und

Y OH, F, Cl, Z-R(5), -OC(O)R(5), -OCO(O)R(5), oder NR(6)R(7), worin

R(5) $(C_1-C_{10})$-Alkyl, $(C_2-C_{10})$-Alkenyl, $(C_3-C_8)$-Cycloalkyl, Phenyl, Benzyl, Methyl-Hetaryl, Biphenylyl, Phenyl-Z-Phenyl, Phenyl-methyl-Phenyl, Naphthyl bedeutet, wobei die aufgeführten aromatischen und heteroaromatischen Ringsysteme unsubstituiert oder mit ein bis drei Substituenten - gleich oder verschieden - aus der Reihe F, Cl, $CF_3$, CN, $(C_1-C_{10})$-Alkyl, $(C_2-C_{10})$-Alkenyl, Z-$(C_1-C_{10})$-Alkyl, Z-$(C_2-C_{10})$-Alkenyl,

| | |
|---|---|
| | $(C_3-C_8)$-Cycloalkyl substituiert ist, |
| R(6) | $(C_1-C_{10})$-Alkyl (geradkettig oder verzweigt, bedeutet und |
| R(7) | $(C_1-C_{10})$-Alkyl (geradkettig oder verzweigt), Phenyl, Benzyl bedeutet und die Phenyl- oder Benzylgruppe neben Wasserstoff 1-3 F-, Cl-Atome oder $CF_3$-, $CH_3$- oder $OCH_3$-Gruppen als Substituenten aufweist, |
| | oder wobei |
| R(6) und R(7) | gemeinsam eine Kette aus $(CH_2)_{4-6}$-Einheiten bilden, in welcher eine $CH_2$-Einheit durch ein Stickstoffatom, welches als zusätzlichen Bindungspartner ein Wasserstoffatom eine Phenyl- oder eine Benzylgruppe trägt, ersetzt sein kann und die Phenyl- oder Benzylgruppe neben Wasserstoff 1-4 F- oder Cl-Atome oder $CF_3$-, $CH_3$- oder $OCH_3$-Gruppen als Substituenten aufweist |
| | und |
| Z | Sauerstoff oder Schwefel ist, und das heteroaromatische Ringsystem ein Heterocyclus aus der Reihe Thiophen, Pyridin, Furan, Oxazol, Isoxazol, Thiazol, Isothiazol ist. |

**3.** Verwendung von Verbindungen I nach Anspruch 1, in denen bedeuten:

| | |
|---|---|
| A | CH, N |
| X | -NR(1)R(2), -O-Phenyl, -O-Benzyl, wobei Phenyl und Benzyl unsubstituiert oder mit ein bis zwei Substituenten aus der Reihe F, Cl, O-$(C_1-C_4)$-Alkyl, $(C_1-C_4)$-Alkyl, substituiert ist), -O-N = CR(3)R(4), worin |
| R(1) | $(C_1-C_{10})$-Alkyl, $(C_2-C_{10})$-Alkenyl, $(C_3-C_6)$-Cycloalkyl, Phenyl, Benzyl, Biphenylyl, Phenyl-Z-Phenyl, Phenyl-methyl-Phenyl ist, wobei die aufgeführten aromatischen Ringsysteme unsubstituiert oder mit ein bis zwei Substituenten - gleich oder verschieden - aus der Reihe F, Cl, $CF_3$, $(C_1-C_{10})$-Alkyl, $(C_2-C_{10})$-Alkenyl, Z-$(C_1-C_{10})$-Alkyl, Z-$(C_2-C_{10})$-Alkenyl, substituiert sind und Z für Sauerstoff oder Schwefel steht |
| | und |
| R(2) | Wasserstoff bedeutet oder wie R(1) definiert ist, wobei R(1) und R(2) gleich oder verschieden sind, |
| | oder |
| R(1) und R(2) | gemeinsam eine Kette von $(CH_2)_{4-5}$-Einheiten bilden, in welcher eine $(CH_2)$-Einheit durch ein Stickstoffatom ersetzt sein kann und wobei das zusätzliche N-Atom als weiteren Bindungspartner ein Phenyl oder Benzyl trägt und der aromatische Rest wie unter R(1) angegeben substituiert sein kann, |
| | oder |
| R(1) und R(2) | gemeinsam mit dem Stickstoffatom, an das sie gebunden sind, einen 1,2,3,4-Tetrahydrochinolinyl- oder 1,2,3,4-Tetrahydroisochinolinyl-Rest bilden, wobei der aromatische Ring unsubstituiert oder mit bis zu zwei Substituenten der Reihe F, Cl, $CF_3$, $CH_3$, $OCH_3$, substituiert sein kann, |
| R(3) | Phenyl, Biphenylyl, Phenylmethylphenyl, Phenyl-Z-Phenyl, Benzyl, $(C_1-C_{10})$-Alkyl ist, wobei die erwähnten aromatischen Ringsysteme unsubstituiert oder mit ein bis zwei Substituenten aus der Reihe F, Cl, $OCH_3$, $(C_1-C_{10})$-Alkyl, $CF_3$, $(C_3-C_6)$-Cycloalkyl, $(C_2-C_8)$-Alkenyl substituiert sind und Z für Sauerstoff oder Schwefel steht, |
| | und |
| R(4) | Wasserstoff, $(C_1-C_{10})$-Alkyl, $(C_2-C_{10})$-Alkenyl, Phenyl, Benzyl bedeutet, wobei der aromatische Ring wie unter R(3) angegeben unsubstituiert oder ein bis zweifach substituiert sein kann, |
| | und |
| Y | OH, F, Cl, Z-R(5), -OC(O)R(5), -OCO(O)R(5), oder NR(6)R(7) worin |
| R(5) | $(C_1-C_{10})$-Alkyl, $(C_2-C_{10})$-Alkenyl, Phenyl, Benzyl, Biphenylyl, Phenyl-Z-Phenyl, Phenyl-methyl-Phenyl, bedeutet, wobei die aufgeführten aromatischen Ringsysteme unsubstituiert oder mit ein bis zwei Substituenten - gleich oder verschieden - aus der Reihe F, Cl, $CF_3$, $(C_1-C_{10})$-Alkyl, $(C_2-C_{10})$-Alkenyl, Z-$(C_1-C_{10})$-Alkyl, Z-$(C_2-C_{10})$-Alkenyl, $(C_3-C_8)$-Cycloalkyl substituiert sind, |
| R(6) | $(C_1-C_{10})$-Alkyl (geradke4ttig oder verzweigt, bedeutet und |
| R(7) | $(C_1-C_{10})$-Alkyl (geradkettig oder verzweigt), Phenyl, Benzyl bedeutet und die |

| | |
|---|---|
| | Phenyl- oder Benzylgruppe neben Wasserstoff 1-3 F- oder Cl-Atome oder $CF_3$-, $CH_3$- oder $OCH_3$-Gruppen als Substituenten aufweist, oder wobei |
| R(6) und R(7) | gemeinsam eine Kette aus $(CH_2)_{4-5}$-Einheiten bilden, in welcher eine $CH_2$-Einheit durch ein Stickstoffatom welches als zusätzlichen Bindungspartner ein Wasserstoffatom eine Phenyl- oder eine Benzylgruppe trägt, ersetzt sein kann und die Phenyl- oder Benzylgruppe neben Wasserstoff 1-2 F- oder Cl-Atome oder $CF_3$-, $CH_3$- oder $OCH_3$-Gruppen als Substituenten aufweist und |
| Z | Sauerstoff oder Schwefel ist. |

**4.** Medikament zur Prophylaxe und zur Behandlung von systemischen Mykosen, dadurch gekennzeichnet, daß es eine wirksame Menge einer Verbindung I nach Anspruch I enthält.

**5.** Methode zum Behandeln von systemischen Mykosen und zur Prophylaxe gegen solche Mykosen, dadurch gekennzeichnet, daß man eine wirksame Menge einer Verbindung I nach Anspruch 1 verabreicht.